Europäisches Patentamt

European Patent Office

Office européen des brevets

(19)

(11) Publication number: **0 024 706**
**B1**

# EUROPEAN PATENT SPECIFICATION

(12)

(45) Date of publication of patent specification: **13.06.84**

(51) Int. Cl.³: **A 61 B 1/00**

(21) Application number: **80105008.9**

(22) Date of filing: **22.08.80**

(54) Endoscope system.

(30) Priority: **23.08.79 JP 107515/79**

(43) Date of publication of application:
**11.03.81 Bulletin 81/10**

(45) Publication of the grant of the patent:
**13.06.84 Bulletin 84/24**

(84) Designated Contracting States:
**CH DE FR GB IT LI NL SE**

(56) References cited:
**US - A - 4 074 234**

**MACHINE DESIGN, vol. 49, no. 25, November 1977 Cleveland US R.T. DANN: "Mating Fiber Optics and Hydraulics", pages 133–137
LASER + ELEKTRO-OPTIK, March 1979 Stuttgart DE T. NISHISAKA et al.: "Ein Holographie-Endoskop für die medizinische Diagnostik", pages 34–35**

(73) Proprietor: **OLYMPUS OPTICAL CO., LTD.
43-2, 2-chome, Hatagaya Shibuya-ku
Tokyo 151 (JP)**

(72) Inventor: **Hattori, Shinichiro
2-14, Takamatsu Toshima-ku
Tokyo (JP)**

(74) Representative: **KUHNEN & WACKER
Patentanwaltsbüro
Schneggstrasse 3-5 Postfach 1729
D-8050 Freising (DE)**

Courier Press, Leamington Spa, England.

## Description

### Background of the invention

This invention relates to an endoscope system having an endoscope and a light source unit.

With the conventional endoscope system, operation instructions, such as air feed, water feed and suction instructions, inputted from the control section of the endoscope are transmitted to a light source unit through a connector having a number of connection wires and connection pins, and a pump and valve provided in the light source unit are controlled by the operation instructions so transmitted. If the number of operation instructions is increased in the conventional endoscope system, the number of connection wires for transmission is increased. As a result, a universal cord between the control section of the endoscope and the connector becomes thicker and thus the flexibility of the universal cord is lowered, making it difficult to operate the endoscope. If the number of connection wires and connection pins are increased, a complicated connection is required. Furthermore, the endoscope system becomes bulkier and complicated.

It is accordingly an object of this invention to provide an endoscope system which can transmit a number of operation instruction signals through a lesser number of transmission paths.

### Summary of the invention

According to this invention there is provided an endoscope system as claimed in claims 1 and 5 respectively.

This invention can be more fully understood from the following detailed description when taken in conjunction with the accompanying drawings.

### Brief description of the drawings

Fig. 1 is a schematic view showing an endoscope system according to one embodiment of this invention; and

Fig. 2 shows an optical fiber transmission circuit as used in an endoscope system according to another embodiment of this invention.

### Detailed description

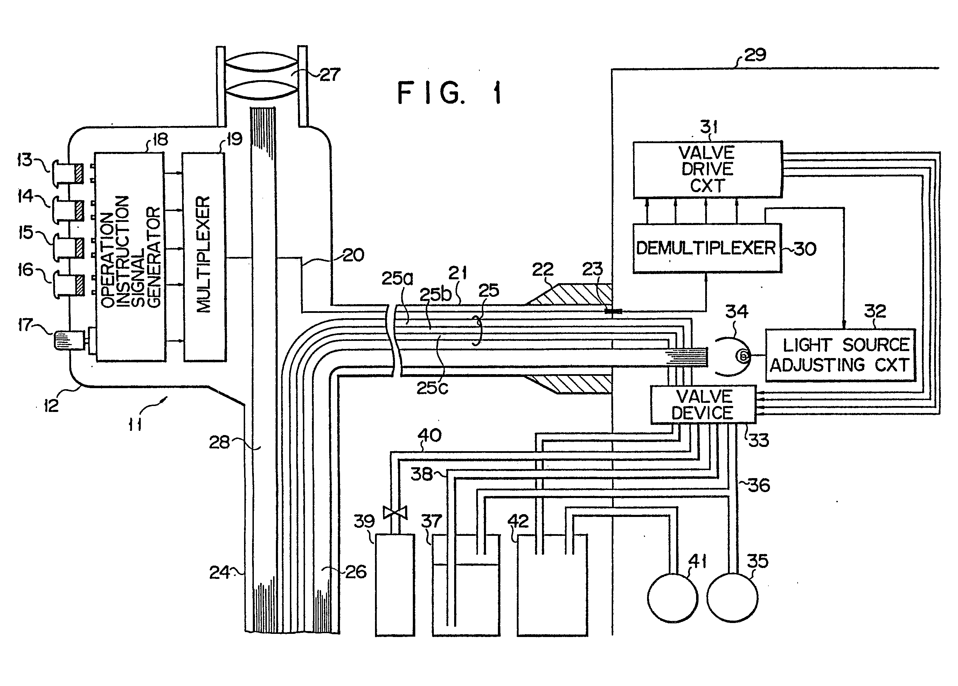

According to the endoscope system as shown in Fig. 1, a light source light quantity adjustor 17 and operation instruction input switches 13, 14, 15 and 16 for inputting operation instructions for an air feed, water feed, suction and air-gas exchange are disposed in the control section 12 of the endoscope 11. The switches 13 to 16 and adjustor 17 are connected to an operation instruction signal generator 18. The operation instruction signal generator 18 has, for example, a read only memory (ROM) stored with operation instruction information corresponding to the air feed, water feed, suction, air-gas exchange and light adjustment. The output terminals of the operation instruction signal generator 18 are connected to a multiplexer 19. The output terminal of the multiplexer 19 is connected to a connection pin 23 on the forward end of the connector 22 through a transmission path 20 which is provided in the universal cord 21. A bundle 25 of tubes such as an air feed tube 25a, water feed tube 25b and suction tube 25c extends from the connector 22 through a universal cord 21 and insertion cord 24 to the distal end (not shown) of an endoscope 11. Likewise, a light guide 26 extends from the connector 22 to the distal end of the endoscope 11. An image guide 28 extends from the distal end of the endoscope 11 to an eyepiece lens system 27. A demultiplexer 30 is provided in a light source unit 29. The input terminal of the demultiplexer 30 is connected to a transmission path 20 through the connection pin 23 of the connector 22 of the endoscope 11. The output terminals of the demultiplexer 30 are connected to a valve drive circuit 31 and light source adjusting circuit 32. The output terminal of the valve drive circuit 31 is connected to a valve device 33 and light source adjusting circuit 32 is connected to a lamp 34. The inlet of the valve device 33 is connected to an air pump 35 through a pipe 36 and to a water tank 37. The water tank 37 is connected through a pipe 38 to the valve device 33 and a $CO_2$ gas bomb 39 is connected through a pipe 40 to the valve device 33. A compressor 41 is connected through a tank 42 to the valve device 33. The outlets of the valve device 33 are connected to the tube bundle 25 of the endoscope 11.

In the above-mentioned system, if the air feed switch 13 is closed, an operation instruction signal generator 18 generates an air feed instruction signal which is read out of ROM. The instruction signal, together with a light amount adjusting signal of the light amount adjustor 17, is supplied to the multiplexer 19. The instruction signal and light adjusting signal are multiplexed by the multiplexer 19 and sent to the transmission path 20. The multiplexed signal on the transmission path 20 is supplied through the connection pin 23 to the demultiplexer 30 in the light source unit 29. The signal is demultiplexed by the demultiplexer 30 into the air feed instruction signal and light adjusting signal. The air feed instruction signal is sent to a magnet valve drive circuit 31. The valve drive circuit 31 supplies a valve drive signal to the valve device 33. The valve device 33 connects the air pump 35 to an air feed tube 25a of the tube bundle 25 of the endoscope 11 in response to the valve drive signal, permitting air to be sent into the body cavity of a human being through the air feed tube 25a. On the other hand, the light adjusting signal controls the light source adjusting circuit 32 to permit the lamp 34 to be lit with a predetermined light quantity. When in this state the air-gas exchange switch 16 is closed, an air-gas exchange instruction signal, together with the air feed signal and light adjusting signal, is multiplexed by the multiplexer 19 and transmitted through the transmission path 20 to the demultiplexer 30 of the light source unit 29. The air-gas exchange instruction signal is demultiplexed by the demultiplexer 30 and supplied to the valve drive circuit 31. The valve drive circuit 31 drives the valve device 33 to permit the pipe 40 of the $CO_2$ gas bomb 39 to be connected to an air feed tube 25a. By so doing, gas from the $CO_2$ gas bomb is supplied to the body cavity of a human being. Now suppose that water is to be supplied. If in this case the switch 14 is closed, a water feed instruction signal is multiplexed by the multiplexer 19 and transmited to the demultiplexer 30. The valve drive circuit 31 drives the valve device 33 based on the water feed instruction signal to permit the water feed pipe 38 to be connected to the water feed tube 25b of the endoscope 11. The water tank 37 receives a pumping pressure from the pump 35 to cause water in the water tank 37 to be pumped through the pipe 38 and valve 33 to a water feed tube 25b. The water injected in the water feed tube 25b is ejected from the end of the insertion cord and cleans the optical member at the end of the insertion cord. When the suction instruction switch 15 is closed after water injection, a suction instruction signal is multiplexed by the multiplexer 19 and transmitted through the transmission path 20 to the demultiplexer 30 in the light source unit 29. As a result, the valve device 33 causes the compressor 41 to be connected to the suction tube 25c of the endoscope 11 to permit water in the body cavity to be sucked out. The sucked out water enters into the tank 42.

In this embodiment, the instruction signals are converted by a parallel-serial converter to a serial mode and the serial signals are subjected to a frequency shift keying (FSK) or is converted to a predetermined frequency signal with the result that it is frequency multiplexed or time-division multiplexed by timing control. The multiplexed signal is transmitted through one coaxial cable or twisted paired wires. According to this invention, therefore, the instruction signals are multiplexed and many instruction signals can be transmitted through one transmission line. As a result, the diameter of the endoscope can be made thinner irrespective of the number of instruction signals. For this reason, the flexibility and operability of the endoscope are improved and thus a quick diagnosis can be made on the endoscope without giving greater pain to the patient. A lesser number of transmission lines permits ready connection and a lesser number of connector pins assures a compact connector. More signals can be transmitted without increasing the number of connector pins. The instruction signals may be analog signals or digital signals or a combination of analog and digital signals.

In this case, the analog signal is subjected to a V-F conversion or FM modulation whereas the digital signal is subjected to a frequency shift keying (FSK). If an LSI for data transmission, a one-chip microcomputer etc. are used as a command signal generator and multiplexer, a control section of the endoscope can be made considerably smaller. As an instruction signal use may be made of a code representing the kinds of the endoscope. The color temperature of the light source and air pressure can be varied according to the kinds of endoscopes. A release signal and exposure control signal from a camera incorporated in the endoscope can be multiplexed and sent to the light source unit.

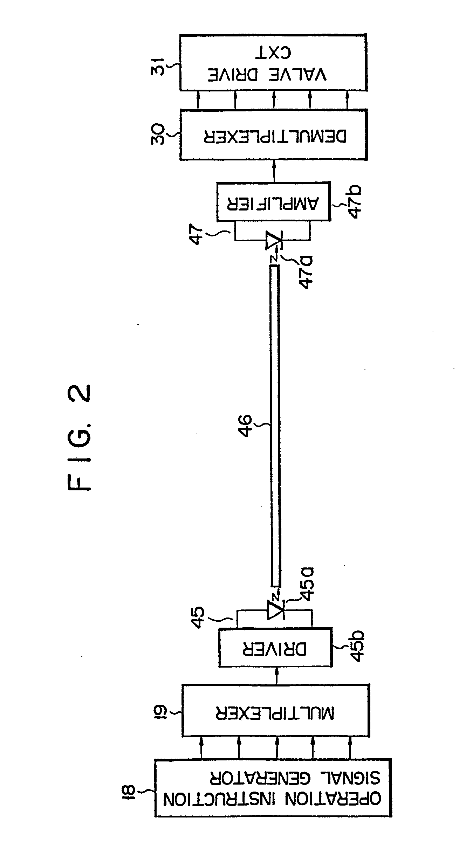

Although in the above-mentioned embodiment a connection wire such as a coaxial cable is used as a transmission line, an optical fiber transmission system may be used as shown in Fig. 2. That is, instruction signals from an instruction signal generator 18 are multiplexed by a multiplexer 19 and sent to a light generating unit 45 including a light-emitting element 45a and a driver 45b. The light generating unit 45 converts the multiplexed signal of the multiplexer 19 to a light signal. The light signal is transmitted through an optical fiber 46 to a light receiving unit 47 including a photoelectric converting element 47a and an amplifier 47b. The output signal of the light receiving unit 47 is supplied to a demultiplexer 30 for demultiplexing. The use of such optical fiber transmission permits a number of signals to be transmitted at higher density.

## Claims

1. An endoscope system having a plurality of operation means for carrying out a plurality of operations, the endoscope system comprising:

an endoscope including a control section; operating means disposed on said control section and which is selectively operable to initiate operations of the endoscope system; and signal generating means (18) coupled to said operating means for generating selected ones of a plurality of signals corresponding to selected operations of said operating means, characterized by including

a universal cord (21) carrying a single signal transmission path (20);

a connector (22) coupled to one end of said universal cord (21);

multiplexing means (19) coupling said signal generating means (18) to said single signal transmission path (20) for multiplexing the signals of said signal generating means (18) into a single multiplexed signal and for feeding said multiplexed signal to said single signal transmission path (20);

said single signal transmission path (20) extending from said multiplexing means (19) to said connector (22) through said universal cord (21) for transmitting said single multiplexed signal of said multiplexing means (19) to said connector (22);

a light source unit (29) coupled to said connector (22) of said endoscope and including demultiplexing means (30) coupled to said single signal transmission path (20) via said connector (22) for demultiplexing said multiplexed signal into individual signals respectively corresponding to the original signals generated by said signal generating means (18); and

driving means coupled to said demultiplexing means (30) for driving at least one of said operation means corresponding to at least one signal of said demultiplexing means (30).

2. An endoscope system according to claim 1, characterized thereby, that said single signal transmission path comprises a coaxial cable.

3. An endoscope system according to claim 1, characterized thereby, that said single signal transmission path comprises optical fiber transmission means.

4. An endoscope system according to anyone of the preceding claims, characterized thereby, that,

said signal generating means (18) includes means for selectively generating at least one of an air feed instruction signal, water feed instruction signal, suction instruction signal and air-gas exchange instruction signal; and said driving means of said light source unit (29) includes a valve device (33) for selectively effecting air feed, water feed, suction and air-gas exchange operations in response to demultiplexed instruction signals.

5. An endoscope system having means for selectively effecting air feed, water feed, suction and air-gas exchange operations; and means for adjusting the brightness of a light source, the endoscope system comprising:

an endoscope including a light guide for guiding an illumination light; an image guide for conducting a light image, a plurality of tubes, operating means which is selectively operable to initiate operations of the endoscope system; and signal generating means coupled to said operating means for selectively generating selected ones of a plurality of operation instruction signals; characterized by including

multiplexing means (19) coupled to said signal generating means (18) for multiplexing the signals generated by said signal generating means (18) into a single multiplexed signal;

a single signal transmission path (20) coupled to said multiplexing means (19) for transmitting said single multiplexed signal of said multiplexing means (19);

a light source unit (29) coupled to said light guide and to said single signal transmission path (20), and including a light source (34) for sending illumination light to said light guide of the endoscope;

demultiplexing means (30) coupled to said single signal transmission path (20) for receiving and demultiplexing said multiplexed signal into individual signals respectively corresponding to the original signals generated by said signal generating means (18);

a valve device (33) coupled to said demultiplexing means and controlled by demultiplexed individual signals of said demultiplexing means for selectively effecting air feed, water feed, suction and air-gas exchange operations;

and means (32) for adjusting the brightness of the light source (34) responsive to a selected individual signal of said demultiplexed signal.

6. An endoscope system according to claim 5, characterized thereby, that said single signal transmission path comprises a coaxial cable.

7. An endoscope system according to claim 5, characterized thereby, that said single signal transmission path comprises optical fiber transmission means.

8. An endoscope system according to claim 1 or 5, characterized thereby, that said multiplexing means (19) multiplexes a plurality of signals generated by said signal generating means (18) into a single multiplexed signal.

**Patentansprüche**

1. Endoskopsystem mit einer Vielzahl von Funktionseinrichtungen zur Ausübung einer Vielzahl von Funktionen, wobei das Endopskopsystem ein Endoskp mit einem Bedienungsabschnitt, Bedienungseinrichtungen, welche in dem Bedienungsabschnitt angeordnet sind und welche wahlweise bedienbar sind, um Funktionen des Endoskopsystems auszulösen, und eine Signalerzeugungseinrichtung (18) aufweist, welche mit den Bedienungseinrichtungen verbunden ist, um aus der Vielzahl von Signalen die Angewählten zu erzeugen, welche mit den angewählten Funktionen der Bedienungseinrichtung übereinstimmen, gekennzeichnet durch:

eine Universalleitung (21), welche einen einzigen Signalübertragungspfad (20) aufweist;

einen Konnektor (22), welcher mit einem Endabschnitt der Unversalleitung (21) verbunden ist;

Multiplex-Einrichtungen (19), welche die Signalerzeugungseinrichtung (18) mit dem einzigen Signalübertragungspfad (20) verbinden, um die Signale der Signalerzeugungseinrichtung (18) in ein einzelnes Multiplex-Signal umzusetzen und um das Multiplex-Signal in den einzigen Signalübertragungspfad (20) einzuspeisen, wobei der einzige Signalübertragungspfad (20) sich von der Multiplex-Einrichtung (19) aus durch die Universalleitung (21) zu dem Konnektor (22) hin erstreckt, um das einzelne Multiplex-Signal von der Multiplex-Einrichtung (19) zu dem Konnektor (22) zu übertragen; eine Lichtquelleneinheit (29), welche mit dem Konnektor (22) des Endoskops verbunden ist und Demultiplex-Einrichtungen (30) aufweist, welche mit dem einzigen Signalübertragungspfad (20) über den Konnektor (22) verbunden sind, um das Multiplex-Signal in einzelne Signale in Übereinstimmung mit den Original-Signalen, welche von der Signalerzeugungseinrichtung (18) erzeugt wurden, zu demultiplexen; und

Treibereinrichtungen, welche mit der Demultiplex-Einrichtung (30) verbunden sind, um wenigstens eine der Funktionseinrichtungen in Übereinstimmung mit wenigstens einem Signal der Demultiplex-Einrichtungen anzutreiben.

2. Endoskopsystem nach Anspruch 1, dadurch gekennzeichnet, daß der einzige Signalübertragungspfad ein Coaxial-Kabel aufweist.

3. Endoskopsystem nach Anspruch 1, dadurch gekennzeichnet, daß der einzige Signalübertragungspfad optische Faserübertragungseinrichtungen aufweist.

4. Endoskopsystem nach einem der vorhergehenden Ansprüche, dadurch gekennzeichnet, daß die Signalerzeugungseinrichtung (18) Einrichtungen aufweist, um wahlweise ein Signal aud der Reiher der Anweisungssignale für Luftzufuhr, Wasserzufuhr, Ábsaugung u. Luft-Gasaustausch zu erzeugen, und daß die Treibereinrichtungen der Lichtquelleneinheit (29) Vintilorgane (33) aufweist, um wahlweise Luftzufuhr-, Wasserzufuhr-, Absaugungsund Luft/Gasaustausch-Funktionen als Antwort auf die Demultiplex-Anweisungssignale auszulösen.

5. Endoskopsystem mit Einrichtungen zur wahlweisen Bedienung von Luftzufuhr, Wasserzufuhr, Absaugung und Luft/Gasaustausch und mit Einrichtungen zum Einstellen der Helligkeit einer Lichtquelle, wobei das Endoskopsystem ein Endoskop mit einer Lichtleitfaser zum Leiten eines Beleuchtungslichtes, eine Bildleitfaser zum Leiten eines Bildlichtes, eine Veilzahl von Röhren, eine Bedienungseinrichtung, mit welcher wahlweise Funktionen des Endoskopsystems auslösbar sind, und Signalerzeugungseinrichtungen, welche mit den Bedienungseinrichtungen verbunden sind, um wahlweise ausgewählte Signale aus der Vielzahl von Funktionsanweisungssignalen auszuwählen, aufweist, gekennzeichnet durch,

Multiplex-Einrichtungen (19), welche mit den Signalerzeugungseinrichtungen (18) verbunden sind, um die Signale, welche von der Signalerzeugungseinrichtung (18) erzeugt weden, in ein einzelnes Multiplex-Signal umzusetzen;

einen einzelnen Signalübertragungspfad (20), welcher mit der Multiplex-Einrichtung (19) verbunden ist, um das einzige Multiplex-Signal von der Multiplex-Einrichtung (19) zu übertragen;

eine Lichtquelleneinheit (19), welche mit der Lichtleitfaser und dem einzigen Signalübertragungspfad (20) verbunden ist, und welche eine Lichtquelle (34) aufweist, um Beleuchtungslicht zu der Lichtleitfaser des Endoskops zu leiten; Demulitplex-Einrichtungen (30), welche mit dem einzigen Signalübertragungspfad (20) verbunden sind, um das Multiplex-Signal zu empfangen und in Einzelsignale umzusetzen, welche mit den Originalsignalen übereinstimmen, welche von der Signalerzeugungseinrichtung (18) erzeugt wurden;

ein Ventilorgan (33), welches mit der Demultiplex-Einrichtung verbunden ist und . von der Demultiplex-Einrichtung umgesetzten Signalen gesteuert wird, um wahlweise Luftzufuhr-, Wasserzufuhr-, Absaugung-, und Luft/Gas-Austauschfunktionen zue verursachen; und

Einrichtungen (32) zur Einstellung der Helligkeit der Lichtquelle (34) in Abhängigkeit von einem ausgewählten Einzelsignal von dem umgesetzten Signal.

6. Endoskopsystem nach Anspruch 5, dadurch gekennzeichnet, daß der einzelne Signalübertragungspfad ein Coaxial-Kabel aufweist.

7. Endoskopsystem nach Anspruch 5, dadurch gekennzeichnet, daß der einzige Signalübertragungspfad optische Faserübertragungseinrichtungen aufweist.

8. Endoskopsystem nach Anspruch 1 oder 5, dadurch gekennzeichnet, daß die Multiplex-Einrichtung (19) eine von der Signalerzeugungseinrichtung (18) erzeugte Vielzahl von Signalen in ein einzelnes Multiplex-Signal umsetzt.

## Revendications

1. Système d'endoscopie comportant plusieurs moyens de fonctionnement pour effectuer une pluralité d'opérations, ce système d'endoscopie comprenant un endoscope incluant une partie de commande, un moyen de fonctionnement disposé sur cette partie de commande et sélectivement manoeuvrable pour déclencher les opérations du système d'endoscopie, et un moyen (18) de création de signaux sélectionnés correspondant aux opérations sélectionnées du moyen de fonctionnement, ce systéme d'endoscopie étant caractérisé en ce qu'il comprend un cordon universel (21) contenant une voie (20) de transmission de signaux uniques; un connecteur (22) couplé à une extrémité de ce cordon universel (21); un moyen (19) de multiplexage couplant le moyen (18) de création de signaux à la voie (20) de transmission de signaux uniques afin de multiplexer les signaux de ce moyen (18) de création de signaux en un seul signal multiplexé et pour introduire ce signal multiplexé dans la voie (20) de transmission de signaux uniques, cette dernière voie (20) allant du moven (19) de multiplexage au connecteur (22) en passant par le cordon universel (21) afin de transmettre le seul signal multiplexé du moyen (19) de multiplexage au connecteur (22); un ensemble (29) à source lumineuse couplé au connecteur (22) de l'endoscope et comprenant un moyen (30) de démultiplexage couplé à la voie (20) de transmission de signaux uniques par l'intermédiaire du connecteur (22) afin de démultiplexer le signal multiplexé en signaux individuels correspondant respectivement aux signaux originaux créés par le moyen (18) de création de signaux; et un moven de commande couplé au moyen (30) de démultiplexage pour commander au moins un des moyens de fonctionnement correspondant à un signal au moins de ce moyen (30) de démultiplexage.

2. Système d'endoscopie suivant la revendication 1, caractérisé en ce que la voie de transmission de signaux uniques comprend un câble coaxial.

3. Système d'endoscopie suivant la revendication 1, caractérisé en ce que le voie de transmission de signaux uniques comprend un moyen de transmission par fibre optique.

4. Système d'endoscopie suivant l'une quelconque des revendications précédentes, caractérisé en ce que le moyen (18) de création de signaux comprend un moyen poure créer sélectivement au moins un des signaux d'instruction d'alimentation en air, d'alimentation en eau, d'aspiration et d'échange air-gaz; et en ce que le moyen de commande de l'ensemble (29) à source lumineuse comprend un dispositif (33) à soupapes pour effectuer sélectivement des opérations d'alimentation en air, d'alimentation en eau, d'aspiration et d'echange air-gaz en réponse à des signaux démultiplexés d'instruction.

5. Système d'endoscopie comportant des moyens pour effectuer sélectivement des opérations d'alimentation en air, d'alimentation en eau, d'aspiration et de'échange air-gaz, et un moyen poure régler la brillance d'une source lumineuse, ce système d'endoscopie comprenant un endoscope incluant un guide le lumière pour guider une lumière d'éclairement, un guide d'image pour conduire une image lumineuse, une pluralité de tubes, un moyen de fonctionnement qui est sélectivement manoeuvrable pour déclencher les opérations du systéme d'endoscopie, et un moyen de création de signaux couplé au moyen de fonctionnement pour créer sélectivement une pluralité de signaux sélectionnés d'instructions de fonctionnement, ce système d'endoscopie étant caractérisé en ce qu'il comprend un moyen (19) de multiplexage couplé au moyen (18) de création de signaux afin de multiplexer les signaux créés par ce moyen (18) de création de signaux en un seul signal multiplexé; une voie (20) de transmission de signaux uniques, couplée au moyen (19) de multiplexage pour transmettre ce seul signal multipexé du moyen (19) de multiplexage; un ensemble (29) à source lumineuse couplé au guide de lumière et à la voie (20) de transmission de signaux uniques, et comprenant une source lumineuse (34) pour envoyer la lumière d'éclairement au guide de lumière de l'endoscope; un moyen (30) de démultiplexage couplé à la voie (20) de transmission de signaux uniques pour recevoir et démultiplexer le signal multiplexé en signaux individuels correspondant respectivement aux signaux originaux créés par le moyen (18) de création de signaux; un dispositif (33) à soupapes couplé au moyen de démultiplexage et commandé par des signaux individuels démultiplexés du moyen ĕe démultiplexage afin d'effectuer sélectivement des opérations d'alimentation en air, d'alimentation en eau, d'aspiration et d'échange air-gaz; et un moyen (32) pour régler la brillance de la source lumineuse (34) en réponse à un signal individuel sélectionné du signal démultiplexé.

6. Système d'endoscopie suivant la revendication 5, caractérisé en ce que la voie de transmission de signaux uniques comprend un câble coaxial.

7. Système d'endoscopie suivant la revendication 5, caractérisé en ce que la voie de transmission de signaux uniques comprend un moyen de transmission par fibre optique.

8. Système d'endoscopie suivant l'une des revendications 1 ou 5, caractérisé en ce que le moyen (19) de multiplexage multiplexe en un seul signal multiplexé une pluralité de signaux créés par le moyen (18) de création de signaux.

FIG. 1

0 024 706

FIG. 2